# EUROPEAN PATENT SPECIFICATION

(11) **EP 2 564 883 B1**
(45) Date of publication and mention of the grant of the patent: **07.05.2014**
(21) Application number: 11179516.7
(22) Date of filing: 31.08.2011
(51) Int. Cl.: A61M 1/10

(54) **Electropneumatic pulsator**
Elektropneumatischer Pulsator
Pulsateur électro-pneumatique

(43) Date of publication of application: 06.03.2013
(73) Proprietor: Fundacja Rozwoju Kardiochirurgii, 41-800 Zabrze (PL)
(72) Inventor: Kustosz, Roman, 41-800 Zabrze (PL); Darlak Maciej, 41-710 Ruda Slaska (PL)
(74) Representative: Malcherek, Piotr

(56) References cited:
- WO-A1-97/14453
- PL-Y1- 65 284
- US-A- 5 217 430
- US-A- 5 300 111
- US-A1- 2009 099 498

## Description

The invention relates to an electro-pneumatic pulsator used in drives of pneumatic ventricular assist devices.

There is a known example of an electro-pneumatic pulsator according to the Polish patent description with publication number PL 181231 B which has a housing comprising two chambers separated by a group of membranes. In one of the chambers there is an electro-pneumatic converter, and in the other chamber there is a pneumatic amplifier. The pneumatic amplifier chamber has three chambers, two of which have inlet connecting pieces for compressed air, and the third mixing chamber located between them has an outlet connecting piece from the mixing chamber for transmitting a pneumatic wave to the ventricular assist device. Between the mixing chamber and the chambers with inlet connecting pieces of compressed air there are two closing valves, supported by a spring and mounted on a pivot. The electro-pneumatic converter converts the transmitted electrical energy into alternate motion of a group of membranes, and said alternate motion of a group of membranes with the compressed air supplied by means of the two inlet connecting pieces results in activating the valves and transmitting the pneumatic wave through the outlet connecting piece of the mixing chamber.

Moreover, there is a known example of artificial heart drive according to the Polish utility model with publication number PL 65284 Y, which has a gas divider with a chamber and two dividing channels. In each of the dividing channels there is a linear motor equipped with a single-acting piston. The pistons of said linear motors divide gas by inlets to pumping units of the artificial heart. There is one inlet in each dividing channel, located in the middle of each side.

Additionally US2009/099498 discloses a cardiopulmonary bypass system utilizing membrane-based reciprocating positive displacement blood pumps ("pod pumps"). In one aspect, the pod pumps are constructed to provide reduced shear forces on the blood being pumped. In another aspect blood flow through the pod pumps can be controlled by a controller using information from pressure sensors in the control chamber of the pod pumps. In another aspect, the pod pumps are included on a disposable unit that can be received and held by a receptacle means on a base unit, the base unit also providing pressurized control fluid to the pod pumps on the disposable unit through the receptacle means.

US 5217430 discloses an apparatus for driving a medical appliance. The apparatus has a single compressor for generating both the positive and the negative pressures. Positive and negative pressure valves for controlling communication with the atmosphere are connected to the outlet and inlet terminals of the compressor and the outlet and inlet terminals of the compressor are also connected to a positive pressure accumulator and a negative pressure accumulator, respectively. The apparatus has pressure sensors to sense when the pressures in the accumulators reach a set pressure. The apparatus further has a one-way valve in the positive pressure line between the compressor and the valve or in the negative pressure line between the compressor and the valve. The apparatus prohibits the positive and negative pressure valves from being open at the same time.

The closest prior art is WO 97/14453. This document discloses an electro-pneumatic pulsator suitable for generating a pneumatic wave for driving pneumatic ventricular assist devices, comprising a body with an internal mixing chamber, two inlet connecting pieces suitable for connecting pneumatic power supply and two regulating valves located coaxially, wherein the first regulating valve is located between the mixing chamber and the first inlet pneumatic connecting piece and the second regulating valve is located between the mixing chamber and the second inlet pneumatic connecting piece, and an outlet pneumatic connecting piece from the mixing chamber for transmitting a pneumatic wave to a ventricular assist device, wherein there is a motor for alternate opening of the regulating valves, and the regulating valves are oppositely oriented and each regulating valve is seated in an assigned pneumatic head.

The invention as claimed differs from the closest prior art in that the motor is a double-acting linear motor arranged between the regulating valves and each of the regulating valves is supported by a spring.

In a preferred embodiment the regulating valves are mushroom valves are located in cone seats of pneumatic heads.

It is also advisable when each of the mushrooms of the valves is guided on a pin located in a barrel in the mushroom axis.

Then, it is recommendable that the pin projects over the frontal surface of the valve mushroom and is a contact element of the mushroom with the piston of the linear motor.

Moreover, it is advisable that both of the inlet pneumatic connecting pieces are coaxial to the regulating valves.

It is also recommendable that in a resting position when both regulating valves are closed the total of distance between the cooperating elements of the first regulating valve and the specifically assigned end of the piston of the linear motor and of the distance between the cooperating elements of the second regulating valve and the specifically assigned second end of the linear motor piston is smaller than the minimum piston stroke of the linear motor.

Electro-pneumatic pulsators are actuators used , for example, in pneumatic drives of ventricular assist devices. The aim of a pulsator is converting a control signal in the form of an electrical signal into a pressure wave and a pneumatic flow with strictly defined parameters depending on the application. In the case of ventricular assist devices the parameters of the pressure wave generated by a pulsator are closely connected with the patient's safety. Lack of control over these parameters may result in ineffectiveness of the assisting process. It may lead to occurrences such as hemolysis, heart failure, etc. The application of an element opening the regulating valves of the linear motor with high precision of reciprocating movement and repeatability of the piston positioning and accurate dynamic parameters, acceleration and linear velocity has made it possible to devise a construction of a new electro-pneumatic pulsator as an actuator generating a pneumatic wave to drive pneumatic assist devices. The construction of a pulsator with a linear motor compared to purely mechanical constructions has allowed to eliminate many problems such as the influence of interference resulting from pressure drops in the pneumatic drive which was at the same time a supply source of an actuator and drive in the to-date constructions. The use of a linear motor has also allowed to resign from membranes used as coupling devices of valves of actuator drives known from the prior state of the art. Owing to the use of a linear motor and appropriate location of regulating valves and inlet connecting pieces the size of the pulsator could be diminished, which is especially beneficial in view of miniaturizing the ventricular assist device. What is more, the proposed distribution of valves in relation to the cooperating ends of linear motor piston ensures uninterrupted work of a pulsator.

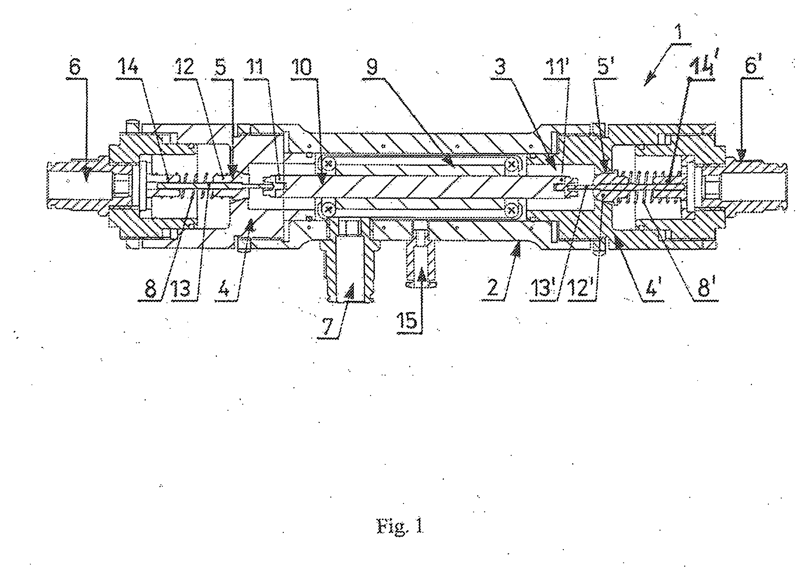

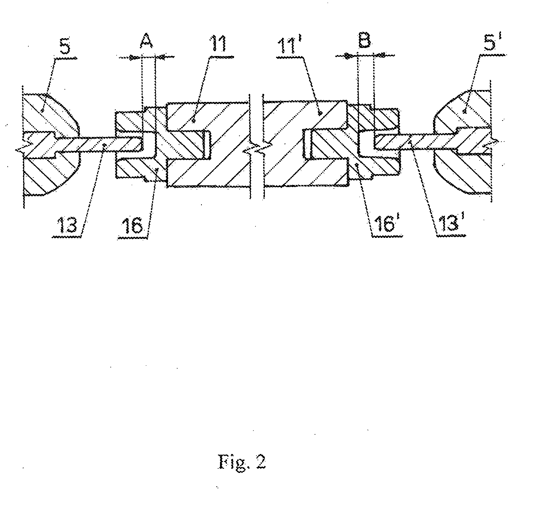

The invention is presented in more detail in the following embodiment and in the attached drawing where fig. 1 shows a longitudinal section of an electro-pneumatic pulsator and fig.2 shows a schematic position of the valves and linear motor piston in a resting position.

An electro-pneumatic pulsator 1 for generating a pneumatic wave for driving pneumatic ventricular assist devices comprises a body 2 with an internal mixing chamber 3. On each side of the body 2 there are pneumatic heads 4 and 4'. Between the head 4 and the mixing chamber 3 there is a regulating valve 5. Between the head 4' and the mixing chamber 3 there is a regulating valve 5'. On the other side the head 4 is equipped with an inlet pneumatic connecting piece 6. The head 4' on the other side is equipped with an inlet connecting piece 6'. The inlet connecting pieces 6 and 6' are used for connecting pneumatic supply and are advantageously located coaxially to the regulating valves 5, 5'. The mixing chamber 3 comprises an outlet pneumatic connecting piece 7 for transmitting a pneumatic wave to a ventricular assist device. The regulating valves 5 and 5' are located coaxially and are oppositely oriented and each regulating valve 5, 5' located in the assigned pneumatic head 4, 4' is supported by a spring 8, 8' which springs 8, 8' close the regulating valve 5, 5' in a resting position. The acting force of the springs, in particular springs 8, 8' of valves 5, 5' is adjustable. Between the regulating valves 5, 5' there is a double-acting linear motor 9 for alternate opening of the regulating valves 5,5'. The double - acting linear motor 9 comprises a piston 10 for single-directional, double-sided work comprising a first end 11 and a second end 11'. The linear motor 9 is mounted on the body 2 by mounting means, for example screws. Electrical wiring not displayed in the drawing is connected to the motor 9, for example by means of an electrical connector not displayed in the drawing, located in the body 2 of the pulsator 1. Moreover, the ends 11, 11' of the piston 10 may comprise sockets with cooperating elements of the valves 5, 5'.

The regulating valves 5, 5' may be mushroom valves located in cone seats of the pneumatic heads 4, 4'. Each of said mushrooms 12, 12' of the valves 5, 5' is guided on a pin 13, 13' located in a barrel 14, 14' located in the axis of said mushroom 12, 12'. The pins 13, 13' project over the frontal surface of the mushroom 12, 12' and are a contact point of said mushroom with ends 11, 11' of the piston 10 of the linear motor 9, for example with the sockets 16, 16' located on ends 11, 11' of the piston 10.

In a resting position where both regulating valves 5, 5' are closed by means of a spring 8, 8', the total of distance A between the cooperating , in particular adjacent elements of first regulating valve 5, in particular end of the pin 13 and the assigned end 11 of the piston 10 of the linear motor 9, in particular the socket 16 mounted on end 11 of the piston 10 and of distance B between the cooperating, in particular adjacent elements of second regulating valve 5', in particular other end of the pin 13' and the assigned end 11' of the piston 10 of the linear motor 9, in particular the socket 16' mounted on end 11' of the piston 10 is smaller than the minimum stroke of the linear motor 9. In practice distances A and B should ideally be as small as possible, close to 0 or equal 0.

In order to achieve effective and reliable operation, the body 2 of the pulsator 1 after closing the inlet pneumatic connecting pieces 6, 6' and the outlet connecting piece 7 is hermetically tight.

The body 2 of the pulsator 1 may be optionally equipped with a connecting piece 15 for measuring pressure inside the mixing chamber 3.

The linear motor 9 is located in the body 2 of the pulsator 1 between two mushroom valves 5,5'. In the resting position the valves 5, 5' are closed and the piston 10 of the motor 9 is in "zero" point, in equilibrium between the two valves 5, 5'. The movement of the piston 10 will result in opening of one of the valves. The degree of opening of each of the valves determines the flow field and is a function of the distance between the mushroom and the socket. The use of a linear motor allows to precisely control time and degree of opening of the valve. Owing to the use of separable fastening between the mushroom and the motor piston, the valves open alternately, which means that when the mushroom of the first valve is open, the mushroom of the second valve remains closed. The mixing chamber is the volume between the two valves, to which air may be introduced by the valve of the first head or the valve of the second head. By connecting the heads of a pulsator to different pneumatic power supply sources, for example vacuum or overpressure and by inducing cyclical work of the linear motor, it is possible to generate a pressure wave of various parameters in the mixing chamber. Said wave may be utilised for driving other pneumatic elements. In a ventricular assist system said wave is utilised for driving pneumatic ventricular device.

The pulsator, due to its hermeticity, may also be used in both closed and open pneumatic systems.

## Claims

1. An electro-pneumatic pulsator ( 1 ) for generating a pneumatic wave for driving pneumatic ventricular assist devices, comprising a body ( 2 ) with an internal mixing chamber ( 3 ), two inlet connecting pieces ( 6, 6' ) for connecting pneumatic power supply and two regulating valves ( 5, 5' ) located coaxially, wherein the first regulating valve ( 5 ) is located between the mixing chamber ( 3 ) and the first inlet pneumatic connecting piece ( 6 ) and the second regulating valve ( 5' ) is located between the mixing chamber ( 3 ) and the second inlet pneumatic connecting piece ( 6' ), and an outlet pneumatic connecting piece ( 7 ) from the mixing chamber ( 3 ) for transmitting a pneumatic wave to a ventricular assist device, wherein between the regulating valves ( 5, 5' ) there is a motor ( 9 ) for alternate opening of the regulating valves ( 5, 5'), and said regulating valves ( 5, 5') are oppositely oriented and each regulating valve (5, 5') is seated in an assigned pneumatic head ( 4, 4'), **characterised in that** the motor ( 9 ) is a dual acting linear motor arranged between the regulating valves ( 5, 5' ) and each regulating valve ( 5, 5' ) is supported by a spring element (8,8').

2. The pulsator according to claim 1 **characterised in that** the regulating valves ( 5, 5') have the form of mushroom valves located in cone seats of the pneumatic heads (4, 4').

3. The pulsator according to claim 1 or 2 **characterised in that** each of the mushrooms ( 12, 12') of the valves ( 5, 5') is guided on a pin (13, 13') located in a barrel (14, 14') located in the axis of the mushroom (12, 12').

4. The pulsator according to claim 3 **characterised in that** the pin (13, 13') projects over the frontal surface of the mushroom ( 12, 12') of the valve ( 5, 5' ) and is a contact element of the mushroom ( 12, 12') with a piston ( 10 ) of the linear motor ( 9 ).

5. The pulsator according to one of claims 1 - 4 **characterised in that** both inlet pneumatic connecting pieces ( 6, 6') are located coaxially to the regulating valves ( 5, 5').

6. The pulsator according to one of claims 1 - 5 **characterised in that** in a resting position where both regulating valves (5,5') are closed, the total of distance (A) between the cooperating elements of first regulating valve (5) and an assigned end (11) of the piston (10) of the linear motor (9) and of the distance between the cooperating elements of the second regulating valve (5') and an assigned second end (11') of the piston (10) of the linear motor (9) is smaller than the minimum stroke of the linear motor (9).

## Patentansprüche

1. Elektropneumatischer Pulsator /1/ für Formung der pneumatischen Welle zum Antreiben von pneumatischen Herz-Hilfspumpen, der das gehäuse /2/ mit der inneren Mischkammer /3/, zwei pneumatischen Eingangsverbindungen /6, 6'/ zum Anschluss der pneumatischen Versorgung sowie zwei koaxial gelegten Regelventile /5, 5'/ aufweist, wobei das erste Regelventil /5/ zwischen der Mischkammer /3/ und der ersten pneumatischen Eingangsverbindung /6/ lokalisiert ist, das zweite Regelventil /5'/ dagegen zwischen der Mischkammer /3/ und der zweiten pneumatischen Eingangsverbindung /6'/ lokalisiert ist, sowie eine aus der Mischkammer /3/ ausgehende pneumatische Ausgangsverbindung /7/ zur Übergabe der pneumatischen Welle an die pneumatische Herz-Hilfspumpe besitzt, wobei zwischen den Regelventilen /5, 5% der Motor /9/ zum abwechselnden Öffnen der Regelventile /5, 5'/ lokalisiert ist, und die Regelventile /5, 5`/ selbst entgegengesetzt orientiert sind, und jeder der Regelventile /5, 5'/ in dem ihm zugeordneten pneumatischen Kopf /4, 4'/ platziert ist, **gekennzeichnet dadurch**, der Motor/9/ein Linearmotor zweiseitiger Wirkung ist, angeordnet zwischen den Regelventilen /5, 5'/, und jeder Regelventil /5, 5'/ mittels dem elastischen Element /8, 8'/ unterstützt ist.

2. Pulsator nach Anspruch 1 **dadurch gekennzeichnet, dass** die Regelventile /5, 5'/ die Form von in Kegelbüchsen der pneumatischen Köpfe /4, 4'/ eingesetzten Tellerventilen ausweisen.

3. Pulsator nach Anspruch 1 oder 2 **dadurch gekennzeichnet, dass** jeder der Teller /12, 12'/ der Ventile /5, 5'/ mittels einer Stecknadel /13,13') geführt ist, welche sich in der Hülse /14, 14'/ befindet, die in Achse des Tellers /12, 12'/ lokalisiert ist.

4. Pulsator nach Anspruch 3 **dadurch gekennzeichnet, dass** die Stecknadel /13, 13'/ über die Stirnoberfläche des Tellers /12, 12'/ des Ventils /5, 5'/ hinausragt und das Kontaktelement des Tellers /12, 12'/ mit dem Kolben /10/ des Linearmotors /9/ bildet.

5. Pulsator nach einem der Ansprüche 1 bis 4 **dadurch gekennzeichnet, dass** die beiden pneumatischen Eingangsverbindungen /6, 6'/ koaxial mit den Regelventilen /5, 5'/ gesetzt sind.

6. Pulsator nach einem der Ansprüche 1 bis 5 **dadurch gekennzeichnet, dass** in der Ruhelage, in der die beiden Regelventile /5, 5'/ geschlossen sind, die Summe der Abstandweite /A/ zwischen miteinander mitwirkenden Elementen des ersten Regelventils /5/ und dem ihm zugeordneten ersten Ende /11/ des Kolbens /10/ des Linearmotors /9/ sowie der Abstandweite zwischen den miteinander mitwirkenden Elementen des zweiten Regelventils /5'/ und dem ihm zugeordneten zweiten Ende /11'/ des Kolbens /10/ des Linearmotors /9/ kleiner als der minimale Hub des Linearmotors /9/ ist.

## Revendications

1. Le pulsateur électropneumatique (1), formant une onde pneumatique pour entraîner les chambres pneumatiques d'assistance cardiaque, comprenant un corps (2) avec une chambre de mélange interne (3), deux connecteurs d'entrée (6, 6') pneumatiques reliant la source d'alimentation pneumatique et deux soupapes régulatrices (5, 5') disposées coaxialement l'une par rapport à l'autre, la première soupape régulatrice (5) étant située entre la chambre de mélange (3) et le premier connecteur d'entrée (6) pneumatique, et la seconde soupape régulatrice (5) étant située entre la chambre de mélange (3) et le second connecteur d'entrée (6') pneumatique, ainsi qu'un connecteur de sortie (7) pneumatique de la chambre de mélange (3) pour amener l'onde pneumatique à la chambre d'assistance cardiaque, où entre lesdites soupapes régulatrices (5, 5') est localisé un moteur (9) ouvrant alternativement les soupapes régulatrices (5, 5'), lesdites soupapes régulatrices (5, 5') étant orientées de manière opposée et chaque soupape régulatrice (5, 5') étant située dans une tête pneumatique associée (4, 4'), **caractérisé en ce que** ce que le moteur (9) est un moteur linéaire à double effet, disposé entre les soupapes régulatrices (5, 5') et chaque soupape régulatrice (5, 5') est supportée par un élément à ressort (8, 8').

2. Le pulsateur selon la revendication 1, **caractérisé en ce que** les soupapes régulatrices (5, 5') sont réalisées sous forme de soupapes en champignon incorporées dans les fentes coniques de têtes pneumatiques (4,4').

3. Le pulsateur selon les revendications 1 ou 2, **caractérisé en ce que** chaque champignon (12, 12') des soupapes (5, 5') est guidé sur une épingle (13, 13') située dans un manchon (14, 4') localisé dans l'axe du champignon (12; 12').

4. Le pulsateur selon la revendication 3, **caractérisé en ce que** l'épingle (13, 13') fait saillie au-dessus de la surface frontale du champignon (12, 12') de la soupape (5, 5') et constitue un élément de contact du champignon (12, 12') avec le piston (10) du moteur linéaire (9).

5. Le pulsateur selon l'une des revendications 1 à 4, caiactérisé en ce que les deux connecteurs d'entrée pneumatiques (6, 6') sont disposés coaxialement par rapport aux soupapes régulatrice (5, 5').

6. Le pulsateur selon l'une des revendications 1 à 5, **caractérisé en ce que** dans la position de repos, où les deux soupapes régulatrices (5, 5') sont fermés, la somme des distances (A) entre les éléments coopérants de la première soupape régulatrice (5) et la première extrémité qui lui est associée (11) du piston (10) du moteur linéaire (9), et des distances entre les éléments coopérants de la seconde soupape régulatrice (5') et la seconde extrémité qui lui est associée (11') du piston (10) du moteur linéaire (9) est inférieure au saut minimum du moteur linéaire (9).
